# EUROPEAN PATENT SPECIFICATION

(11) **EP 2 020 434 B1**
(45) Date of publication and mention of the grant of the patent: **10.10.2012**
(21) Application number: 07425509.2
(22) Date of filing: 03.08.2007
(51) Int. Cl.: C12M 1/107, E02B 15/04

(54) **Reactor for the anaerobic production of biogas**
Reaktor zur anaeroben Erzeugung von Biogas
Reacteur pour la production anaérobie de biogaz

(43) Date of publication of application: 04.02.2009
(73) Proprietor: ACEA Pinerolese Industriale S.p.A., 10064 Pinerolo (IT)
(72) Inventor: Dal Col Adriano, 10064 Pinerolo (IT)
(74) Representative: Bergadano, Mirko

(56) References cited:
- EP-A- 0 520 172
- BE-A1- 897 693
- US-A- 3 954 619

## Description

The present invention relates to a reactor for the anaerobic production of biogas from pre-treated wet waste.

There are known biogas production plants comprising a feeding station of wet waste in bags, a pre-treatment unit of the aforesaid wet waste, and a reactor, in which the pre-treated wet waste biochemically reacts with appropriate micro-organisms. Such biochemical reaction generates biogas useable as source of energy and a reject, known as digestate, meant to be conveyed to a composting plant.

More specifically, the pre-treatment unit sequentially performs on the wet waste a first crushing adapted to break the bags, a sieving adapted to eliminate predetermined impurities, and a second crushing contemplated to take the wet waste itself to a sufficiently small dimension to be made suitable for the subsequent biochemical reaction.

Bio-digestion plants further comprise a tank interposed between the pre-treatment unit and the reactor along the wet waste feed line.

More specifically, such tank is adapted to dilute in water and heat up the wet waste coming from the pre-treatment unit, concurrently separating a fraction of non-biodegradable discards from the wet waste itself.

The reactor essentially comprises a first chamber receiving the water and wet waste mixture from the tank, and in which the inert materials are eliminated from the aforesaid mixture by precipitation; and a second chamber, in which the aforesaid mixture is made to react, under anaerobic conditions, with micro-organisms generating the biogas and the digestate.

More specifically, the second chamber surrounds the first chamber and receives the aforesaid mixture, by overflowing, from the first chamber itself.

The biogas predominantly consists of methane and for the remaining part of carbon dioxide and other components, and it is accumulated in a storage gasometer, from where it is subsequently conveyed to a use station, e.g. a co-generation plant.

A first fraction of the digestate is accumulated, by gravity, in a bottom portion of the second chamber, from where it is subsequently conveyed, after having been dehydrated, to a composting plant.

A second fraction of the digestate, lighter than the aforesaid first fraction, floats on the free surface of the wet waste and water mixture which is reacting with the micro-organisms in the second chamber.

Such second fraction is manually removed, in a known manner, i.e. by creating a manhole for an operator in a region of the second chamber adjacent to the aforesaid free surface and employing an appropriate manual tool, with which the operator manually removes the second fraction and collects it in a discharge tank.

It is felt in the sector the need to automate the second fraction removal operation so as to make the removal operation itself more cost-effective, efficient and repeatable.

It is also felt in the sector the need to automate the removal operation without introducing a number of components and elements such as to increase the complexity of the reactor.

BE-A-897693 discloses a reactor according to the preamble of claim 1. US-A-3954619 discloses a reactor comprising a movable element to convey the floating solids towards an outlet.

It is the object of the present invention to make a reactor for the anaerobic production of biogas from pre-treated wet waste, which allows to simply and cost-effectively fulfil the aforesaid need.

The aforesaid object is reached by the present invention in that it relates to a reactor for the anaerobic production of biogas from pre-treated wet waste as defined in claim 1.

For a better understanding of the present invention, a preferred embodiment will be described below, only by way of non-limitative example and with reference to the accompanying figures, in which:
- figure 1 is a functional diagram of an anaerobic digestion plant of pre-treated wet waste comprising a reactor according to the present invention with parts removed for clarity;
- figure 2 shows an axial section of the reactor of the plant in figure 1;
- figure 3 is a top view of the reactor in figure 2; and
- figure 4 shows a highly enlarged perspective view of some details of the reactor in figures 2 and 3.

With reference to the accompanying figures, numeral 1 indicates a pre-treated wet waste anaerobic bio-digestion plant.

In greater detail, the plant 1 essentially comprises:
- a pre-treatment unit 2 (only diagrammatically shown) receiving pre-treated wet waste in bags;
- a tank 3 adapted to dilute the pre-treated wet waste in water and heat it up to approximately 65 degrees centigrade; and
- a reactor 4 receiving a wet waste and water mixture exiting from the tank 3, and in which such mixture reacts with appropriate micro-organisms generating biogas and a reject, known as digestate.

Specifically, the reactor 4 discontinuously processes fair volumes of the aforesaid mixture, i.e. receives in input a volume of mixture, and discharges such volume, once the mixture has completed the reaction with the micro-organisms.

Furthermore, a heavy fraction of the digestate precipitates, by gravity, inside the reactor 4, while a light fraction of the digestate itself floats on a free surface 61 of the reacting wet waste and water mixture.

Such light fraction of the digestate essentially consists of organic component particles.

Inside the unit 2, the wet waste undergoes a pre-treatment essentially consisting of a first, course crushing aimed at tearing the bags, of a sieving adapted to deprive the wet waste from predetermined impurities and of a second crushing adapted to make the wet waste of sufficiently small dimensions to be able to react with the micro-organisms in the reactor.

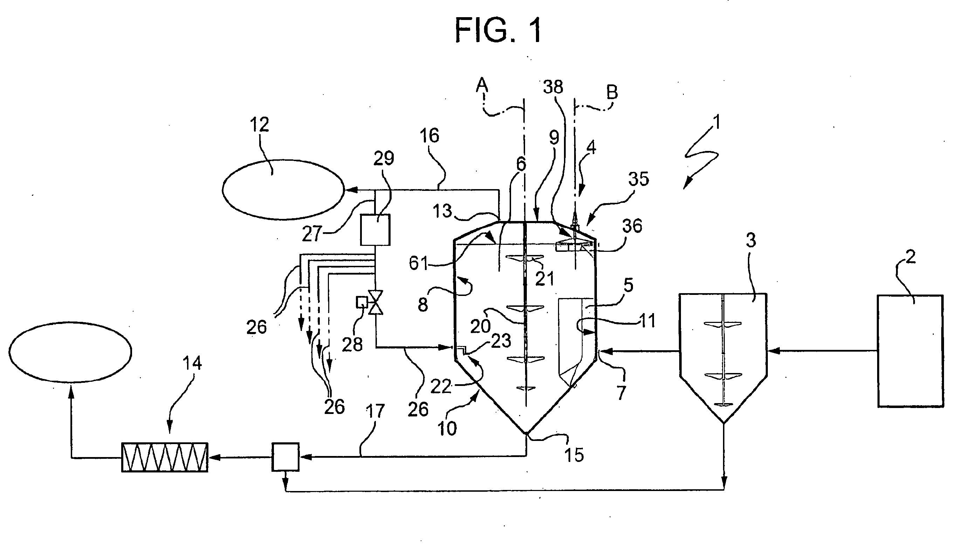

The tank 3 is adapted to maintain the wet waste thus pre-treated in a state of stirring (in a manner only diagrammatically shown in figure 1) so as to separate a fraction consisting of non-biodegradable discards from the wet waste and water mixture.

Such wet waste and water mixture, after having left the tank 3, is pumped (in a manner not shown) into the reactor 4.

The reactor 4 (figures 1, 2 and 3) essentially comprises:
- a pre-chamber 5 receiving the waste and water mixture from the tank 3 by means of an inlet mouth 7 and adapted to separate, by precipitation, a fraction of inert materials from the aforesaid mixture; and
- a reaction chamber 6 surrounding the pre-chamber 5, receiving the waste and water mixture from the pre-chamber 5 itself, and in which such mixture biochemically reacts under anaerobic conditions with the micro-organisms generating biogas and digestate.

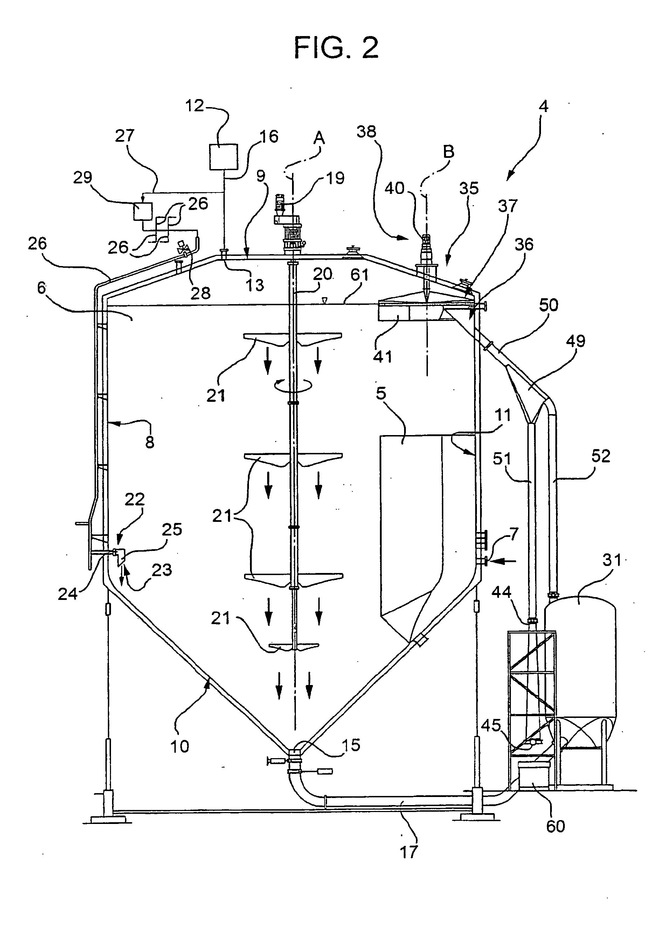

In greater detail (figure 2), the reactor 4 extends along an axis A vertically arranged in use and is delimited by a main cylindrical wall 8 of axis A, by a top wall 9 and by a truncated cone bottom wall 10 having decreasing radial dimension proceeding from the wall 8 opposing the wall 9.

The walls 9, 10 are arranged on opposite sides of the wall 8.

The wall 8 laterally delimits the chamber 6 with respect to axis A, and faces away from axis A, while the walls 9, 10 are incident and transversal to axis A.

The pre-chamber 5 protrudes overhanging in the reactor 4 from a segment 11 of the wall 8 and from a segment, contiguous to the segment 11, of the wall 10.

Furthermore, the pre-chamber 5 extends in the reactor 4 in an eccentric position with respect to axis A and on one side of axis A itself.

The pre-chamber 5 essentially comprises a closed bottom meant to collect the inert materials separated by precipitation, and a side wall extending from the segment 11 of the wall 8.

The segment 11 of the wall 8 is crossed by the inlet mouth 7, which is fluidically connected to the tank 3 to allow the water and wet waste mixture to reach the pre-chamber 5 itself.

Finally, the pre-chamber 5 is open in the upper part and on the side opposite to the closed bottom to allow the wet waste and water mixture to overflow into the chamber 6.

The chamber 6 occupies the volume of the reactor 4 not occupied by the pre-chamber 5, is selectively connectable to a biogas storage gasometer 12 (only diagrammatically indicated in figures 1 and 2) by means of an outlet mouth 13 obtained in the wall 9, and is selectively connectable to a treatment plant 14 (only diagrammatically indicated in figure 1) of the heavy fraction of the digestate, by means of an outlet mouth 15 obtained at an end of the wall 10, opposite to the wall 8.

More specifically, a conveying pipe 16 of the biogas is interposed between the outlet mouth 13 and the gasometer 12, and a conveying pipe 17 of the heavy digestate fraction is interposed between the outlet mouth 15 and the plant 14.

The biochemical reaction occurs at a temperature of approximately 50 degrees centigrade and the wet waste and water mixture remains in the chamber 6 itself for at least fourteen days.

Furthermore, the reactor 4 comprises a stirring system interacting with the wet waste and water mixture to maintain the mixture itself in a state of constant motion preventing the partial solidification thereof, and to promote the progress of the reaction with the micro-organisms.

The stirring system comprises a shaft 20 accommodated in the chamber 6, turnable about axis A, and provided with a plurality of disc-shaped appendixes 21, four in the case in point, radially protruding from the shaft 20 itself and interacting with the wet waste and water mixture.

The shaft 20 is actuated by a motor 19 (shown only in figure 2), extends overhanging from the wall 9 and presents a free end opposite to the wall 9 itself, accommodated in the chamber 6 and surrounded by the wall 10.

The stirring system comprises supplying means of a plurality of biogas jets in the chamber 6.

More specifically, the supplying means are fed with the biogas produced in the chamber 6, pass through the wall 8 and are provided with a plurality of supplying mouths 23, five in the case in point (only one of which is shown in figure 2), of corresponding biogas jets in the chamber 6.

The mouths 23 are arranged in corresponding portions adjacent to the wall 8 to maintain corresponding regions of the mixture spaced from the shaft 20 and adjacent to the wall 8 itself in a continuous state of stirring.

In greater detail, the supplying means comprise:
- a plurality of pipes 26, five in the case in point, connected to the pipe 16 and each presenting a main portion extending externally to the chamber 6 and an end portion crossing the wall 8; and
- a plurality of nozzles 22 (only one of which is shown in figures 1 and 2) provided with corresponding mouths 23, each fluidically connected to a corresponding pipe 26 and adapted to supply the corresponding biogas jets inside the chamber 6.

In greater detail, each pipe 26 is fluidically interposed between a corresponding deviation 27 of the pipe 16 and the corresponding nozzle 22.

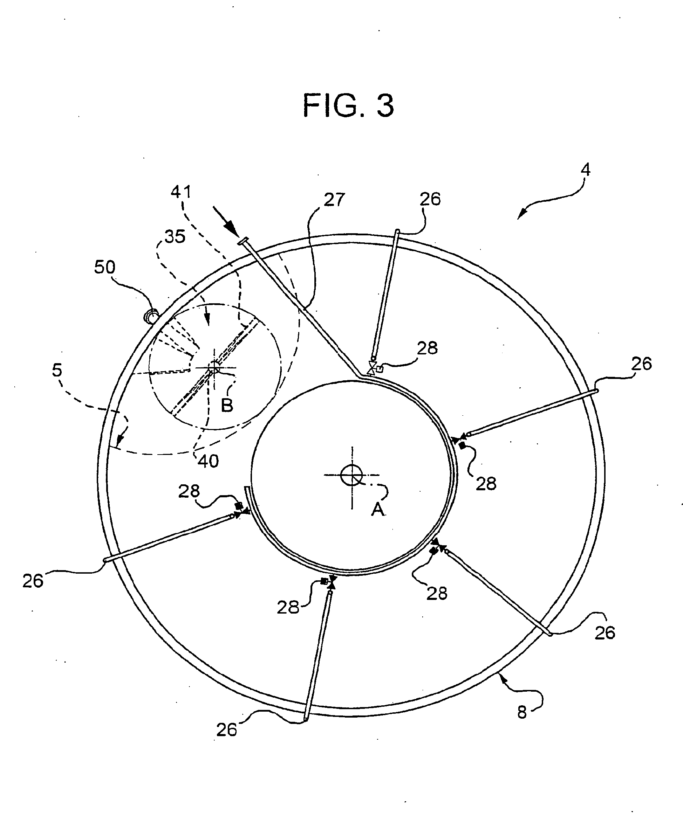

Along such deviation 27, there is arranged a compressor 29 (only diagrammatically shown in figures 2 and 3) adapted to increase the pressure of the biogas fed to pipes 26 and supplied into chamber 6.

The pipes 26 are secured to the external surfaces of the walls 8 and 9.

Along the pipes 26 there are interposed corresponding valves 28 (only one of which is shown in figures 1 and 2) electronically controlled by a control unit (not shown in the accompanying figures).

More specifically, the valves 28 are controlled so that only one of them is opened, while the other valves 28 remain closed.

The opening sequence of the valves 28 depends on the viscosity of the water and wet waste mixture and is such to promote a displacement action of the aforesaid mixture towards the wall 9.

By controlling the valves 28 so that the biogas flows only along one pipe 26 at a time, and thus so that only one mouth 23 at a time supplies biogas into chamber 6, the formation of a circular crown of solidified wet waste on the internal surface of the chamber 6 is also prevented.

Each nozzle 22 is arranged inside the chamber 6, and comprises a first end receiving the biogas from the corresponding pipe 26, and a second free end, opposite to the first end, defining the outlet mouth 23 of the corresponding biogas jet.

Furthermore, each nozzle 22 comprises a first segment 24 extending in a radial direction with respect to axis A and provided with the corresponding first end, and a second segment 25 extending from the segment 24 parallelly to axis A and towards the outlet mouth 15. The segment 25, moreover, defines the mouth 23.

The biogas jets exiting from the mouths 23 are thus directed parallelly to axis A, towards the wall 10 and in a position adjacent to the wall 8. In such manner, such jets maintain the wet waste and water mixture in motion in corresponding regions of the chamber 6 adjacent to the wall 8 and away from the shaft 20.

Specifically, each mouth 23 lays on an inclined plane with respect to axis A and is shaped so as to move closer to the wall 9, proceeding from the corresponding pipe 26 towards the shaft 20.

Such shape of the mouths 23 slows down the speed of the biogas passing through the mouths 23 themselves and contrasts the occlusion of the mouths 23 by the reacting water and wet waste mixture.

Furthermore, the reactor 4 comprises a tank 31 fluidically connected to the chamber 6 and crossed by the pipe 17. The tank 31, being fluidically connected to the chamber 6, is normally full of biogas.

Advantageously, the reactor 4 comprises a removal device 35 of the light fraction of the digestate accommodated in the chamber 6 and comprising, in turn:
- a collection element 36 of the light fraction, fluidically connectable to a discharge tank 60 (shown in figure 2) of the light fraction itself, and
- a mobile conveying element 38 adapted to interact with the light fraction at the free surface 61 to convey the light fraction itself towards the collection element 36.

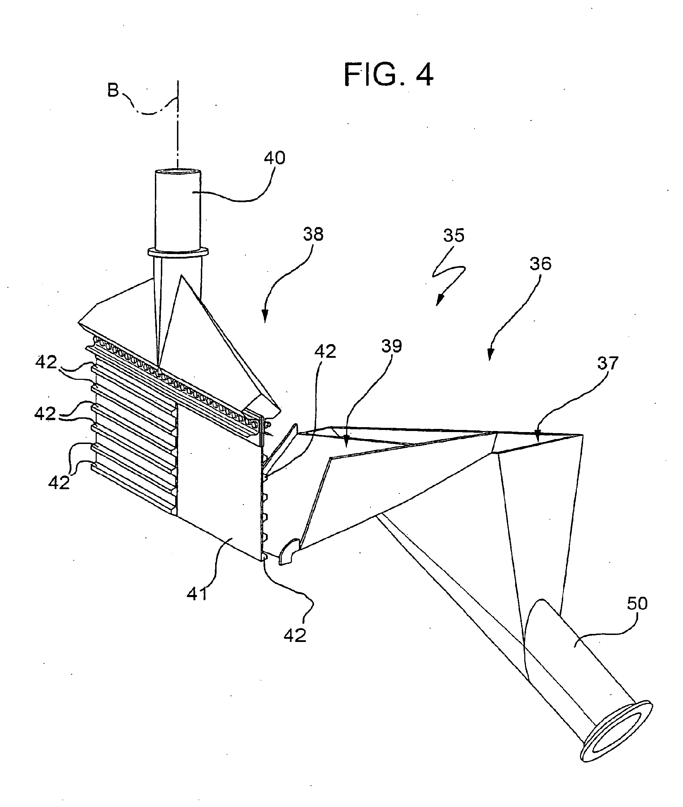

The device 35 (figure 4) is arranged in an eccentric position with respect to axis A and higher than the appendix 21 arranged in the highest position and thus, closest to the wall 9. Furthermore, the device 35 is essentially arranged at the same distance as the pre-chamber 5 from the axis A.

The element 36 is fixed to the reactor 4 and comprises a basin 37 open towards the wall 9 and fluidically connected, on the side opposite to the wall 9, to the discharge tank 60.

More precisely, the element 36 comprises a wall defining a chute 39 adjacent to the element 38, inclined with respect to axis A and immersed in the wet waste and water mixture immediately underneath the free surface 61.

The chute 39 presents a first end edge contiguous to the basin 37 and a second free end edge, opposite to the first end.

Furthermore, the first edge of the chute 39 extends for a shorter length than the second edge of the chute 39 itself.

The chute 39 thus defines a feed surface for the light fraction of the digestate conveyed from the element 38 itself towards the basin 37 converging towards the basin 37 itself.

Specifically, the first edge of the chute 39 is arranged in a higher position than the second edge of the chute 39 itself so as to promote the return of the water dragged by the element 38 along with the light fraction of the reject back into the chamber 6.

The basin 37 further presents a pipe 50 fluidically connected to the discharge tank 60 on the opposite side of the wall 9.

The element 38 is rotational about an axis B, parallel to axis A, to carry away such light fraction and guide it along the chute 39 and into the basin 37.

The element 38 specifically comprises a motor (not shown), a shaft 40 operatively connected to the motor itself to be rotationally fed about axis B, and a crossbar 41 integral with the shaft 40 and provided, on both sides, with a plurality of strips 42 of elastically deformable material, specifically rubber, adapted to be immersed (figures 1 and 2) immediately underneath the free surface 61 and to interact with the light floating fraction to guide it along the chute 39 and towards the basin 37.

The crossbar 41 lays on a plane parallel to axis B and vertically arranged in use, while the strips 42 are elongated orthogonally to axis B.

The crossbar 41 is delimited in the lower part by an edge opposite to the shaft 40 and arranged at the same height as the second edge of the chute 39.

The pipe 50 presents a first end connected to the basin 37 and a second end opposite to the first end defined by a branch 49. A tube 52 ending in the tank 31 and a tube 51 ending in the discharge tank 60 of the light fraction of the digestate originate at such branch 49.

Furthermore, the tube 51 presents a pair of reciprocally spaced valves 44, 45 adapted to discharge the light fraction of the digestate without generating leakages of biogas.

More specifically, the valve 44 is interposed between the valve 45 and the branch 49.

The tube 51 is normally full of biogas and of the light fraction of the digestate. Specifically, the light fraction of the digestate is heavier than the biogas and is arranged, by gravity, at the end adjacent to the valve 44 of the portion of the tube 51 interposed between the branch 49 and the valve 44.

The valve 44 is, moreover, displaceable between an opened position in which it allows the passage of the light fraction of the digestate into the portion of the tube 51 interposed between the valves 44, 45, and a second closed position in which it prevents the biogas contained in the tube 51 from reaching the portion of the tube 51 interposed between the valves 44, 45.

The valve 45 is arranged in a closed position when the valve 44 is arranged in the opened position so as to trap the light fraction of the digestate in the portion of the tube 51 comprised between the valves 44, 45.

Otherwise, the valve 45 is arranged in the opened position, when the valve 44 is arranged in the closed position to allow the light fraction of the digestate previously trapped between the valves 44, 45 to reach the discharge tank 60.

In use, the unit 2 performs a plurality of pre-treatments on the wet waste fed in bags. More specifically, such pre-treatments comprise a first crushing aimed at tearing the bags, a sieving adapted to deprive the wet waste of predetermined impurities, and a second crushing contemplated to make the wet waste of sufficiently small dimensions to be able to react with the micro-organisms in the reactor 4.

The wet waste exiting from the unit 2 reach the tank 3, where they are diluted and heated up to approximately sixty-five degrees centigrade and, later, they advance towards the inlet 7 of the pre-chamber 5.

Inside the pre-chamber 5, the inert materials are separated from the mixture and precipitate onto the closed bottom of the pre-chamber 5 itself.

The wet waste and water mixture overflows from the pre-chamber 5 into the chamber 6, where it reacts, at a temperature of approximately 50-55 degrees centigrade and for a time of at least fourteen days, with the micro-organisms present in the chamber 6 itself generating biogas and digestate.

The biogas moves up towards the wall 9, while the heavy fraction of the digestate precipitates by gravity inside the chamber 6 and accumulates on the wall 10.

Otherwise, the light fraction of the biogas floats on a free surface 61 of the wet waste and water mixture.

After the reaction is completed, the outlet mouth 13 is opened and the biogas reaches the gasometer 12 by means of the pipe 16. The biogas is stored in the gasometer 12 and subsequently made available to a co-generation plant.

By means of the appendixes 21, the rotation of the shaft 20 maintains the region of the wet waste and water mixture adjacent to axis A in a constant state of stirring.

Furthermore, part of the biogas which flows along the pipe 16 is deviated along the deviation 27, compressed by the compressor 29 and made available to the pipes 26.

Each pipe 26 supplies the pressurized biogas to the corresponding nozzle 22, which expels a corresponding biogas jet by means of the corresponding mouth 23 in proximity of a corresponding segment of the wall 8 and in the direction of the wall 10.

Such biogas jets contribute to maintain the wet waste and water mixture which is reacting in the chamber 6 at corresponding regions adjacent to the wall 8 and spaced from the shaft 20 in a state of continuous motion.

Furthermore, the valves 28 are controlled by the control unit so that the pressurized biogas can flow along one only pipe 26 and be introduced into the chamber 6 by means of only one nozzle 22.

The nozzle 22 through which the biogas is supplied into the chamber 6 is selected so as to promote the displacement of the reacting wet waste and water mixture towards the device 35, and to contrast the formation of a circular crown of solidified mixture on the internal surface of the reactor 4.

Specifically, by alternating the nozzle 22 from which the biogas jet exits, such circular crown is broken as soon as it is formed.

When it is necessary to remove the light fraction from the free surface 61, the shaft 40 turns about axis B so that the crossbar 41 pushes the light fraction of the digestate along the chute 39 inside the basin 37. The light fraction is later conveyed to the discharge tank 60 by means of the pipe 50 and the tube 51.

When the light fraction of the digestate is discharged, the valves 44, 45 are both closed so that the light fraction may occupy the segment adjacent to the valve 44 of the portion of the tube 51 interposed between the branch 49 and the valve 44.

More specifically, the light fraction of the digestate, arranging adjacent to the valve 44, pushes the biogas into the segment adjacent to the branch 49 of the portion of the tube 51 interposed between the branch 49 and the valve 44.

Afterwards, the valve 44 is opened so at to allow the light fraction of the digestate to occupy the segment of the tube 51 interposed between the valves 44, 45.

The opening times of the valve 44 are such that most of the biogas remains in the portion of the tube 51 comprised between the branch 49 and the valve 44.

Finally, the valve 44 is closed and the valve 45 is opened allowing to supply the light fraction of the digestate into the discharge tank, containing in this manner the risk of leakages of biogas into the atmosphere.

At the end of the biochemical reaction, the heavy fraction of the digestate is discharged by means of the outlet mouth 15, passes through the pipe 17 and fills the tank 31.

The discharge operation of the heavy fraction of the digestate determines an overpressure in the pipe 17 and in the tank 31, thus elevating the pressure of the biogas contained in the tank 31 itself.

In virtue of the fact that the tank 31 is fluidically connected, by means of the tube 52 and the pipe 50, to the chamber 6, such overpressure is balanced returning the biogas back into the chamber 6 itself.

From an examination of the features of the reactor 4 according to the invention, the advantages that it allows to obtain are apparent.

The reactor 4, in virtue of the presence of the device 35, allows to automatically and efficiency remove the light fraction of the digestate without needing to employ an operator and to contemplate a manhole for allowing such operator to reach the free surface 61 of the wet waste and water mixture.

Furthermore, the device 35 removes such light fraction of the digestate efficiently, continuously and repeatedly in time.

Moreover, the reactor 4 according to the present invention allows to reach the aforesaid advantages in a particularly simple manner without being particularly complex in terms of construction.

Indeed, in virtue of the branch 49, the tube 50 is employed both to fluidically connect the tank 31 and the chamber 6 and to fluidically connect the basin 37 and the tube 51 connected to the discharge tank 60.

In such manner, the need to create a tube specifically dedicated to connecting the basin 37 to the discharge tank 60 is avoided.

Finally, the reactor 4, in virtue of the valves 44, 45 and their opening law, allows to limit the entity of the leakage of biogas during the step of conveying of the light fraction of the digestate to the discharge tank, thus limiting the danger of sparks and fires.

It is finally apparent that changes and variants can be made to the reactor 4 previously described without departing from the scope of protection of the present invention.

Specifically, the reactor 4 could comprise a single nozzle 22 and a single pipe 26.

Furthermore, the end portion of the pipes 26 could be outside the reactor 4 and the nozzle 22 could cross the wall 8 of the reactor 4 itself.

## Claims

1. A reactor (4) for the anaerobic production of biogas from pre-treated wet waste, comprising a reaction chamber (6) feedable with a mixture comprising said pre-treated wet waste and adapted to house a biochemical reaction in anaerobic environment; said biochemical reaction transforming said mixture into biogas and into a reject comprising a first fraction, meant to precipitate in said chamber (6), and a second fraction, lighter than said first fraction, meant to float on a free surface (61) of said mixture;
said reactor (4) further comprising:
- a shaft (20) accommodated in said chamber (6), turnable about a first axis (A) and provided with a plurality of appendixes (21) radially protruding from said shaft (20) and interacting with, in use, said mixture;
- a first motor (19) actuating, in use, said shaft (20);
- removal means (35) of the second fraction itself accommodated inside said chamber (6);
said removal means (35) comprising:
- a collection element (36) of the second fraction fluidically connectable to a storage station (60) of said second fraction; and
- a mobile conveying element (38) adapted to interact with said second fraction at said free surface (61) to convey said second fraction itself towards said collection element (36);
said collection element (36) defining a basin (37) fluidically connectable to said storage station (60); said conveying element (38) being rotational about a second axis (B) and comprising a crossbar (41) adapted to interact with said second floating fraction to push it towards said basin (37);
**characterized in that** said conveying element (38) comprises:
- a second motor distinct from said first motor (19); and
- a second shaft (40) rotational about said second axis (B), operatively connected to said second motor, and integral with said crossbar (41);
said second axis (B) being distinct from said first axis (A);
said conveying element (38) being distinct from said appendixes (21).

2. A reactor according to claim 1, **characterised in that** said collection element (36) defines a sliding surface (39) for said second fraction contiguous to said basin (37), adjacent to said crossbar (36) and inclined with respect to said second axis (B).

3. A reactor according to claim 1 or 2, **characterised in that** said crossbar (41) presents a laying plane parallel to said second axis (B).

4. A reactor according to any preceding claim, **characterised in that** said crossbar (41) comprises at least one strip (42) of elastically deformable material arranged on an external surface of said crossbar (41); said strip (42) being adapted to be immersed immediately underneath said free surface (61) and to interact with said second fraction to push it towards said collection element (36).

5. A reactor according to claim 4, **characterised in that** said strip (42) is elongated along a direction transversal to said second axis (B).

6. A reactor according to any preceding claim, **characterised in that** it comprises a further shaft (20) rotational in said chamber (6) about said first axis (A) and interacting with said mixture to maintain it in a state of continuous motion in said chamber (6); and **in that** said removal means (35) are eccentrically arranged with respect to said first axis (A).

7. A reactor according to any preceding claim, **characterised in that** said collection element (36) is fixedly supported by the reactor (4) itself.

8. A reactor according to any preceding claim, **characterised in that** it comprises a first pipe (50) connected, at a first end thereof, to said basin (37) so as to be able to receive said biogas and said second fraction from said chamber (6); said first pipe (50) being connected, at a second end (49) thereof, opposite to said first end and defining a branch (49) of the first pipe (50) itself, to a collection tank (31) of said first fraction of said reject; furthermore, said first pipe (50) being fluidically connectable at said branch (49), with said storage station (60).

9. A reactor according to claim 8, **characterised in that** it comprises:
- a second pipe (51) fluidically connected, at said branch (49), with said first pipe (50) to receive said biogas and said second fraction of said reject, and fluidically connectable, on the side opposite to said branch (49), to said storage station (60); and
- a first valve (45) arranged along said second pipe (51) and a second valve (44) interposed along said second pipe (51) between said first valve (45) and said branch (49) of the first pipe (50); said second valve (44) being selectively available in a first position, in which it allows the passage of said second fraction into the region of said second pipe (51) interposed between said first and second valves (45, 44), and in a second position, in which it prevents said biogas from reaching said region interposed between said first and second valves (44, 45); said first valve (45) preventing the passage of said second fraction towards said storage station (60) when said second valve (44) is in said first position and allowing such passage when said second valve (44) is in said second position.

## Patentansprüche

1. Reaktor (4) für die anaerobe Erzeugung von Biogas aus vorbehandeltem feuchten Abfall, der eine Reaktionskammer (6) aufweist, die mit einer Mischung belieferbar ist, welche den feuchten Abfall aufweist, und die dazu ausgelegt ist, eine biochemische Reaktion in einer anaeroben Umgebung unterzubringen, wobei die biochemische Reaktion die Mischung in Biogas und in einen Rückstand transformiert, der eine erste Fraktion, die in der Reaktionskammer (6) ausgefällt werden soll, und eine zweite Fraktion aufweist, die leichter ist als die erste Fraktion und die auf einer freien Oberfläche (61) der Mischung schwimmen soll;
wobei der Reaktor (4) ferner folgendes aufweist:
- eine Welle (20), die in der Reaktionskammer (6) untergebracht ist, um eine erste Achse (A) drehbar ist und mit einer Vielzahl von Ansätzen (21) versehen ist, die von der Welle (20) radial vorstehen und die, im Betrieb, mit dem Gemisch zusammenwirken;
- einen ersten Motor (19), der, im Betrieb, die Welle (20) betätigt;
- eine Beseitigungseinrichtung (35) für die zweite Fraktion, die im Innenraum der Reaktionskammer (6) untergebracht ist;
wobei die Beseitigungseinrichtung (35) folgendes aufweist:
- ein Sammelelement (36) für die zweite Fraktion, das fluidmäßig an eine Lagerstation (60) für die zweite Fraktion anschließbar ist; und
- ein bewegliches Förderelement (38), das dazu ausgelegt ist, mit der zweiten Fraktion an der freien Oberfläche (61) zusammenzuwirken, um die zweite Fraktion selbst zu dem Sammelelement (36) hin zu fördern;
wobei das Sammelelement (36) ein Becken (37) bildet, das fluidmäßig an die Lagerstation (60) anschließbar ist; wobei das Förderelement (38) um eine zweite Achse (B) drehbar ist und eine Querstrebe (41) aufweist, die dazu ausgelegt ist, mit der schwimmenden zweiten Fraktion zusammenzuwirken, um diese zu dem Becken (37) hin zu bewegen,
**dadurch gekennzeichnet,**
**dass** das Förderelement (38) folgendes aufweist:
- einen zweiten Motor getrennt von dem ersten Motor (19); und
- eine zweite Welle (14), die um die zweite Achse (B) drehbar ist, die betriebsmäßig mit dem zweiten Motor verbunden ist und die integral mit der Querstrebe (41) ausgebildet ist;
wobei die zweite Achse (B) von der ersten Achse (A) getrennt ist und wobei das Förderelement (38) getrennt von den Ansätzen (21) ist.

2. Reaktor nach Anspruch 1,
**dadurch gekennzeichnet,**
**dass** das Sammelelement (36) für die zweite Fraktion eine Gleitfläche (39) bildet, welche an das Becken (37) anschließt, der Querstrebe (36) benachbart ist und gegenüber der zweiten Achse (B) geneigt ist.

3. Reaktor nach Anspruch 1 oder 2,
**dadurch gekennzeichnet,**
**dass** die Querstrebe (41) eine Ebene parallel zu der zweiten Achse (B) bildet.

4. Reaktor nach einem der vorhergehenden Ansprüche,
**dadurch gekennzeichnet,**
**dass** die Querstrebe (41) mindestens einen Streifen (42) aus elastisch deformierbarem Material aufweist, der an einer äußeren Oberfläche der Querstrebe (41) angebracht ist, wobei der Streifen (42) dazu ausgelegt ist, unmittelbar unterhalb der freien Oberfläche (41) einzutauchen und mit der zweiten Fraktion zusammenzuwirken, um diese zu dem Sammelelement (36) hin zu drücken.

5. Reaktor nach Anspruch 4,
**dadurch gekennzeichnet,**
**dass** der Streifen (42) langgestreckt längst einer Richtung ist, die quer zu der zweiten Achse (B) verläuft.

6. Reaktor nach einem der vorhergehenden Ansprüche,
**dadurch gekennzeichnet,**
**dass** er eine weitere Welle (20) aufweist, die in der Reaktionskammer (6) um die erste Achse (A) drehbar ist und mit der Mischung zusammenwirkt, um sie in einem Zustand kontinuierlicher Bewegung in der Reaktionskammer (6) zu halten;
und **dass** die Beseitigungseinrichtung (35) exzentrisch bezüglich der ersten Achse (A) angeordnet ist.

7. Reaktor nach einem der vorhergehenden Ansprüche,
**dadurch gekennzeichnet,**
**dass** das Sammelelement (36) fest von dem Reaktor (4) selbst gelagert ist.

8. Reaktor nach einem der vorhergehenden Ansprüche,
**dadurch gekennzeichnet,**
**dass** er eine erste Leitung (50) aufweist, die an ihrem ersten Ende mit dem Becken (37) verbunden ist, so dass sie in der Lage ist, das Biogas und die zweite Fraktion aus der Reaktionskammer (6) aufzunehmen;
**dass** die erste Leitung (50) an ihrem zweiten Ende, welches dem ersten Ende gegenüberliegt und eine Abzweigung (49) der ersten Leitung (50) selbst bildet, mit einem Sammeltank (31) für die erste Fraktion des Rückstands verbunden ist; und dass die erste Leitung (50) fluidmäßig an der Abzweigung (49) an die Lagerstation (60) anschließbar ist.

9. Reaktor nach Anspruch 8,
**dadurch gekennzeichnet,**
**dass** er folgendes aufweist:
- eine zweite Leitung (51), die an der Abzweigung (49) fluidmäßig an die erste Leitung (50) anschließbar ist, um das Biogas und die zweite Fraktion des Rückstands aufzunehmen, und die fluidmäßig auf der gegenüberliegenden Seite der Abzweigung (49) an die Lagerstation (60) anschließbar ist; und
- ein erstes Ventil (45), das längs der zweiten Leitung (51) angeordnet ist, und ein zweites Ventil (44), das längs der zweiten Leitung (51) zwischen dem ersten Ventil (45) und der Abzweigung (49) der ersten Leitung (50) angeordnet ist;
wobei das zweite Ventil (44) selektiv zur Verfügung steht in einer ersten Position, in der es den Durchgang der zweiten Fraktion in den Bereich der zweiten Leitung (51) zwischen dem ersten Ventil (45) und dem zweiten Ventil (44) ermöglicht, und in einer zweiten Position, in der es verhindert, dass das Biogas den Bereich zwischen dem ersten Ventil (45) und dem zweiten Ventil (44) erreicht; wobei das erste Ventil (45) den Durchgang der zweiten Fraktion zu der Lagerstation (60) hin verhindert, wenn sich das zweite Ventil (44) in der ersten Position befindet, und einen derartigen Durchgang ermöglicht, wenn sich das zweite Ventil (44) in der zweiten Position befindet.

## Revendications

1. Réacteur (4), pour la production anaérobie de biogaz à partir de déchets humides prétraités, comprenant une chambre de réaction (6), susceptible d'être alimentée avec un mélange comprenant lesdits déchets humides prétraités et adaptée pour héberger une réaction biochimique dans un milieu anaérobie ; ladite réaction biochimique transformant ledit mélange en biogaz et en un rejet comprenant une première fraction, destinée à précipiter dans ladite chambre (6), et une deuxième fraction, plus légère que ladite première fraction, destinée à flotter sur une surface libre (61) dudit mélange ;
ledit réacteur (4) comprenant en outre :
- un arbre (20), logé dans ladite chambre (6), susceptible de tourner autour d'un premier axe (A) et muni d'une pluralité d'appendices (21) faisant saillie radialement dudit arbre (20) et interagissant, en utilisation, avec ledit mélange ;
- un premier moteur (19), actionnant, en utilisation, ledit arbre (20) ;
- des moyens d'enlèvement (35) de la deuxième fraction, eux-mêmes, logés à l'intérieur de ladite chambre (6) ;
lesdits moyens d'enlèvement (35) comprenant :
- un élément de collecte (36) de ladite deuxième fraction, susceptible d'être connecté fluidiquement à une station de stockage (60) de ladite deuxième fraction ; et
- un élément de transport (38) mobile, adapté pour interagir avec ladite deuxième fraction à ladite surface libre (61), pour transporter ladite deuxième fraction elle-même vers ledit élément de collecte (36) ;
ledit élément de collecte (36) définissant un bassin (37), susceptible d'être connecté fluidiquement à ladite station de stockage (60) ; ledit élément de transport (38) étant susceptible de tourner autour d'un deuxième axe (B) et comprenant une traverse (41), adaptée pour interagir avec ladite deuxième fraction flottante, de manière à la pousser vers ledit bassin (37) ;
**caractérisé en ce que** ledit élément de transport (38) comprend :
- un deuxième moteur, distinct dudit premier moteur (19) ; et
- un deuxième arbre (40), susceptible de tourner autour dudit deuxième axe (B), relié fonctionnellement audit deuxième moteur et solidaire de ladite traverse (41) ;
ledit deuxième axe (B) étant distinct dudit premier axe (A) ;
ledit élément de transport (38) étant distinct desdits appendices (21).

2. Réacteur selon la revendication 1, **caractérisé en ce que** ledit élément de collecte (36) définit une surface de glissement (39) pour ladite deuxième fraction, contiguë audit bassin (37), adjacente à ladite traverse (36) et inclinée par rapport audit deuxième axe (B).

3. Réacteur selon la revendication 1 ou 2, **caractérisé en ce que** ladite traverse (41) présente un plan de pose parallèle audit deuxième axe (B).

4. Réacteur selon l'une quelconque des revendications précédentes, **caractérisé en ce que** ladite traverse (41) comprend au moins une bande (42) en matériau déformable élastiquement, agencée sur une surface externe de ladite traverse (41) ; ladite bande (42) étant adaptée pour être immergée immédiatement au-dessous de ladite surface libre (61) et pour interagir avec ladite deuxième fraction, de manière à la pousser vers ledit élément de collecte (36).

5. Réacteur selon la revendication 4, **caractérisé en ce que** ladite bande (42) est allongée dans une direction transversale audit deuxième axe (B).

6. Réacteur selon l'une quelconque des revendications précédentes, **caractérisé en ce qu'**il comprend un autre arbre (20), monté à rotation dans ladite chambre (6) autour dudit premier axe (A) et interagissant avec ledit mélange de manière à le maintenir en un état de déplacement continu dans ladite chambre (6) ; et **en ce que** lesdits moyens d'enlèvement (35) sont agencés de manière excentrique par rapport audit premier axe (A).

7. Réacteur selon l'une quelconque des revendications précédentes, **caractérisé en ce que** ledit élément de collecte (36) est supporté rigidement par le réacteur (4) lui-même.

8. Réacteur selon l'une quelconque des revendications précédentes, **caractérisé en ce qu'**il comprend un premier tube (50), relié, à une première extrémité de celui-ci, audit bassin (37), de manière à être en mesure de recevoir ledit biogaz et ladite deuxième fraction en provenance de ladite chambre (6) ; ledit premier tube (50) étant connecté, à une deuxième extrémité (49) de celui-ci, opposée à ladite première extrémité et définissant une branche (49) du premier tube (50) lui-même, à un réservoir de collecte (31) de ladite première fraction dudit rejet ; en outre, ledit premier tube (50) étant susceptible d'être relié fluidiquement, à ladite branche (49), à ladite station de stockage (60).

9. Réacteur selon la revendication 8, **caractérisé en ce qu'**il comprend :
- un deuxième tube (51), relié fluidiquement, à ladite branche (49), audit premier tube (50) pour recevoir ledit biogaz et ladite deuxième fraction dudit rejet, et susceptible d'être relié fluidiquement, sur le côté opposé à ladite branche (49), avec ladite station de stockage (60) ; et
- une première soupape (45), agencée le long dudit deuxième tube (51), et une deuxième soupape (44), interposée le long dudit deuxième tube (51), entre ladite première soupape (45) et ladite branche (49) du premier tube (50) ; ladite deuxième soupape (44) étant disponible sélectivement en une première position, dans laquelle elle permet le passage de ladite deuxième fraction dans la région dudit deuxième tube (51) interposé entre lesdites première et deuxième soupapes (45, 44), et en une deuxième position, dans laquelle elle empêche ledit bio gaz d'atteindre ladite région interposée entre lesdites première et deuxième soupapes (44, 45) ; ladite première soupape (45) empêchant le passage de ladite deuxième fraction vers ladite station de stockage (60), lorsque ladite deuxième soupape (44) se trouve en ladite première position, et permettant un tel passage, lorsque ladite deuxième soupape (44) se trouve à ladite deuxième position.
